(19) **Europäisches Patentamt / European Patent Office / Office européen des brevets**

(11) **EP 2 981 214 B1**

(12) # EUROPEAN PATENT SPECIFICATION

(45) Date of publication and mention
of the grant of the patent:
**29.03.2023 Bulletin 2023/13**

(21) Application number: **14778643.8**

(22) Date of filing: **07.04.2014**

(51) International Patent Classification (IPC):
**A61B 7/02** $^{(2006.01)}$     **A61B 8/08** $^{(2006.01)}$
**G16H 50/30** $^{(2018.01)}$     **A61B 8/14** $^{(2006.01)}$
**A61B 5/00** $^{(2006.01)}$     **A61B 5/318** $^{(2021.01)}$

(52) Cooperative Patent Classification (CPC):
**A61B 7/02; A61B 8/5207; A61B 8/5223;
G16H 50/30;** A61B 5/318; A61B 5/7203; A61B 8/14

(86) International application number:
**PCT/KR2014/002951**

(87) International publication number:
**WO 2014/163443 (09.10.2014 Gazette 2014/41)**

(54) **ELECTRONIC STETHOSCOPE APPARATUS AND METHOD OF USING THE SAME**

ELEKTRONISCHE STETHOSKOPVORRICHTUNG UND VERFAHREN ZU IHRER VERWENDUNG

STÉTHOSCOPE ÉLECTRONIQUE ET SON PROCÉDÉ D'UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **05.04.2013   US 201361808699 P
25.04.2013   US 201361815928 P
04.04.2014   KR 20140040719**

(43) Date of publication of application:
**10.02.2016   Bulletin 2016/06**

(73) Proprietor: **Samsung Electronics Co., Ltd.
Suwon-si, Gyeonggi-do 16677 (KR)**

(72) Inventors:
 • **LEE, Yoon-jae**
  **Seoul 152-764 (KR)**
 • **KIM, Young-tae**
  **Seongnam-si**
  **Gyeonggi-do 463-956 (KR)**
 • **KIM, Seoung-hun**
  **Hwaseong-si**
  **Gyeonggi-do 445-757 (KR)**
 • **RYU, Jong-youb**
  **Suwon-si**
  **Gyeonggi-do 441-768 (KR)**

(74) Representative: **Appleyard Lees IP LLP
15 Clare Road
Halifax HX1 2HY (GB)**

(56) References cited:
 **WO-A1-2006/062704      KR-A- 20090 079 006
 US-A- 5 539 831         US-A1- 2003 009 108
 US-A1- 2007 113 649     US-A1- 2008 232 604
 US-A1- 2010 042 003     US-A1- 2013 041 278**

 • **JATUPAIBOON N ET AL: "Electronic stethoscope prototype with adaptive noise cancellation", 2010 8TH INTERNATIONAL CONFERENCE ON ICT AND KNOWLEDGE ENGINEERING, IEEE, PISCATAWAY, NJ, USA, 24 November 2010 (2010-11-24), pages 32-36, XP031853383, ISBN: 978-1-4244-9874-1**
 • **D. DELLA GIUSTINA ET AL: "Embedding a Multichannel Environmental Noise Cancellation Algorithm into an Electronic Stethoscope", INTERNATIONAL JOURNAL OF CIRCUITS, SYSTEMS AND SIGNAL PROCESSING, vol. 5, no. 2, 1 January 2011 (2011-01-01) , pages 184-191, XP055317046,**

## Description

### Technical Field

The present invention relates to an electronic stethoscope apparatus, and more specifically to an electronic stethoscope apparatus which detects bioacoustics by removing noise efficiently, an automatic diagnostic device and method.

### Background Art

Since an electronic stethoscope apparatus uses a microphone to detect bioacoustics, there may be a high possibility of detecting noise as well according to the sensitivity of the microphone. Especially, this noise occurs frequently when a chest piece is rubbed against the body, and the extraneous noise of the chest piece may be detected.

This occurrence of noise affects the accuracy of diagnosis, thus a method of removing noise properly is demanded.

Meanwhile, bioacoustics has different signal features according to the body part and it is possible to diagnose disease when analyzing these signal features. In addition, so far, diseases have been diagnosed separately by using an ultrasonic device, a purse wave device, and an electrocardiography device, and now it is possible to make a more detailed diagnosis of a disease through merging several devices. Therefore, the techniques of measuring bio acoustic and diagnosing diseases by considering information of other devices are requested.

Patent application publication WO2006062704A1 discloses a method and system for monitoring physiological parameters as useful for remote auscultation of the heart and lungs. The system includes an acoustic sensor that has a stethoscopic cup. A membrane is positioned adjacent to a first end of the stethoscopic cup, and an impedance matching element is positioned adjacent to the membrane. The element provides for acoustic impedance matching with a body such as a human torso. A microphone is positioned near the other end of the stethoscopic cup so as to detect sounds from the body. A signal-conditioning module is then operatively connected to the acoustic sensor, and a wireless transceiver is operatively connected to the signal-conditioning module. Auscultation can then occur at a remote facility that receives signals sent from the transceiver. The system may be incorporated in a wearable harness or a vest, and may include accelerometers in addition to acoustic sensors and signal conditioning modules. The accelerometers can assist in conditioning the system to filter noise associated with rapid gross movement of a person wearing the system.

## Disclosure of Invention

### Technical Problem

Exemplary embodiments of the present invention overcome the above disadvantages and other disadvantages not described above. Also, the present invention is not required to overcome the disadvantages described above, and an exemplary embodiment of the present invention may not overcome any of the problems described above.

In view of the foregoing problems, one object of the present invention is to provide an electronic stethoscope apparatus which can remove noise properly.

Another object of the present invention is to provide a technique of measuring bioacoustics and a technique of diagnosing diseases by considering information of other devices.

### Solution to Problem

The invention provides an electronic stethoscope apparatus as set out in claim 1 and a method of using an electronic stethoscope apparatus as set out in claim 4. Optional features are set out in the dependent claims.

To achieve the objects, an electronic stethoscope apparatus according to one embodiment of the present invention includes a bioacoustics detecting part which detects bioacoustics, a noise detecting part which detects noise occurred during the process of detecting bioacoustics, and a noise removal part which outputs the bioacoustics after removing the detected noise from the bioacoustics

At this time, the bioacoustics detecting part may include a microphone.

In addition, the noise detecting part includes a microphone and is installed in the chest piece of the electronic stethoscope apparatus. The noise removal part may output the detected bioacoustics by filtering with the frequency of the noise signal detected by the microphone.

Furthermore, the noise detecting part includes a movement sensor which senses the movement of the electronic stethoscope apparatus. The noise removal part calculates the noise signal by using sensing value which is output from the movement sensor and may output the detected bioacoustics by filtering with the frequency of the noise signal.

An electronic stethoscope apparatus according to another embodiment, not forming part of the present invention, includes a bioacoustics detecting part which detects bioacoustics, an electrocardiography signal detecting part which detects an electrocardiography signal, and a noise removal part which uses the detected electrocardiography signal to estimate the location of the cardiac sound from the detected bioacoustics and removes the noise from the bioacoustics.

At this time, the electrocardiography signal detecting part may detect the feature of the detected electrocardiography signal, and the noise removal part may estimate the location of the cardiac sound from the detected bioacoustics by using the feature of the detected electrocardiography signal.

Furthermore, an electronic stethoscope apparatus according to another exemplary embodiment, not forming part of the present invention, includes: a bioacoustics sensing part for sensing bioacoustics; a purse wave signal detecting part for detecting a purse wave signals; and a purse wave velocity calculation part for measuring the purse wave transfer velocity by using the distance and time between the first location of the sensed bioacoustics and the second location of the detected purse wave signal.

In addition, an automatic diagnostic device according to another exemplary embodiment, not forming part of the present invention, includes: a bioacoustics sensing part for sensing bioacoustics; an ultrasonic image configuration part for irradiating an ultrasonic signal onto a body part and configuring an ultrasonic image by sensing the reflected ultrasonic signal; and an automatic diagnostic part for diagnosing diseases by using the sensed bioacoustics and the configured ultrasonic image.

The method of using an electronic stethoscope apparatus according to one exemplary embodiment of the present invention includes the steps of: sensing bioacoustics; sensing noise in the bioacoustics sensing process; and outputting the sensed bioacoustics after removing the sensed noise from the sensed bioacoustics

At this point, the noise sensing step is performed by using a microphone.

In addition, the microphone is mounted on a chest piece of the electronic stethoscope apparatus, and the sensed bioacoustics may be output by filtering with a frequency of the sensed noise signal through the microphone in the noise removing step.

Furthermore, the noise sensing step senses noise by using a movement sensor for sensing the movement of the electronic stethoscope apparatus, and the noise removing step calculates a noise signal by using sensing value which is output from the movement sensor and outputs the sensed bioacoustics by filtering with the frequency of the noise signal.

Moreover, an automatic diagnostic method according to other exemplary embodiment, not forming part of the present invention, includes the steps of: sensing bioacoustics; detecting an electrocardiography signal; and removing noise from the sensed bioacoustics by estimating the location of a heart sound from the sensed bioacoustics by using the detected electrocardiography signal.

At this point, the electrocardiography signal detecting step may detect the features of the detected electrocardiography signal, and the noise removing step may estimate the location of the heart sound from the sensed bioacoustics by using the features of the detected electrocardiography signal.

In addition, an automatic diagnostic method according to another exemplary embodiment, not forming part of the present invention, in the automatic stethoscope method, includes the steps of: sensing bioacoustics; detecting a purse wave signal; and estimating a purse wave transfer velocity by using the distance and time between the first location of the sensed bioacoustics and the second location of the detected purse wave signal.

## Advantageous Effects of Invention

According to various exemplary embodiments as above, the present invention provides the electronic stethoscope apparatus for removing noise properly.

In addition, the present disclosure provides the technique of estimating bioacoustics and the technique of diagnosing diseases by also considering information of other devices.

## Brief Description of Drawings

The above and/or other aspects of the present invention will be more apparent by describing certain exemplary embodiments of the present invention with reference to the accompanying drawings, in which:

FIG. 1 is a block diagram illustrating a configuration of an electronic stethoscope apparatus according to an exemplary embodiment of the present invention.

FIG. 2 to FIG. 5 are block diagrams illustrating a configuration of an electronic stethoscope apparatus according to various exemplary embodiments, wherein figs. 2 and 3 illustrate embodiments in accordance with the present invention.

FIG. 6 is a reference diagram illustrating the method of separating target bioacoustics from bioacoustics where a plurality of source bioacoustics are synthesized.

FIG. 7 is a block diagram illustrating a configuration of an electronic stethoscope apparatus according to other exemplary embodiment not forming part of the present invention.

FIG. 8 is a block diagram of a configuration of an electronic stethoscope apparatus according to another exemplary embodiment not forming part of the present invention.

FIG. 9 is a block diagram illustrating a block diagram illustrating a configuration of an electronic stethoscope apparatus according to another exemplary embodiment not forming part of the present invention.

FIG. 10 is a reference diagram schematically illustrating an operation of an electronic stethoscope apparatus according to another exemplary embodiment not forming part of the present invention.

FIG. 11 is a drawing illustrating a wave form of an electrocardiography signal.

FIG. 12 is a drawing illustrating a location correlation between a stethoscope signal and an electrocardiography signal.

FIG. 13 is a block diagram illustrating a configuration of an electronic stethoscope apparatus according to another exemplary embodiment not forming part of the present invention.

FIG. 14 is a reference diagram schematically illustrating an operation of an electronic stethoscope apparatus according to another exemplary embodiment not forming part of the present invention.

FIG. 15 is a drawing illustrating wave forms of a heart sound and a purse wave.

FIG. 16 is a block diagram illustrating an operation of an electronic stethoscope apparatus according to another exemplary embodiment not forming part of the present invention.

FIG. 17 is a reference diagram schematically illustrating an operation of an electronic stethoscope apparatus according to another exemplary embodiment not forming part of the present invention

FIG. 18 is a block diagram illustrating a configuration of a remote diagnosis system according to an exemplary embodiment not forming part of the present invention.

FIG. 19 is a flowchart illustrating a remote diagnosis method.

FIG. 20 to FIG. 23 are flowcharts of an automatic diagnostic method.

**Best Mode for Carrying out the Invention**

**Mode for the Invention**

Hereinafter, description will be given in detail of the present disclosure with reference to the accompanying drawings.

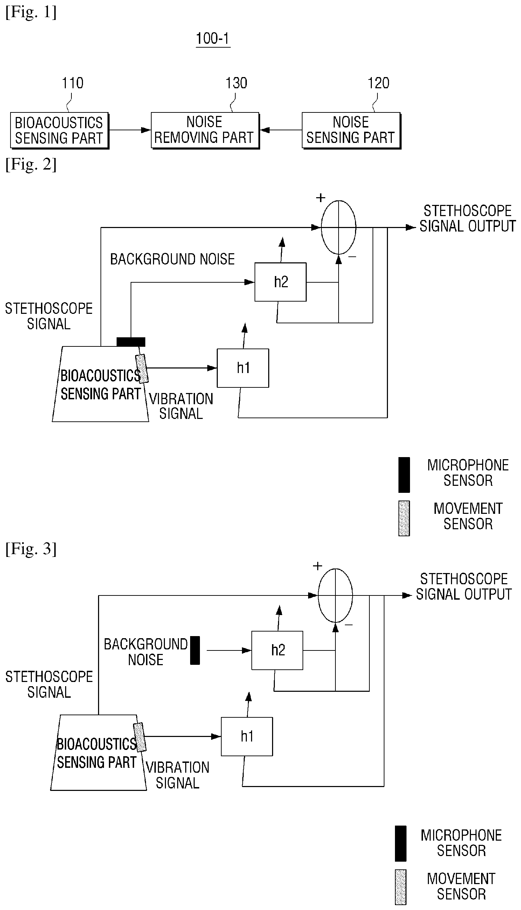

FIG. 1 is a block diagram illustrating a configuration of an electronic stethoscope apparatus 100-1 according to an exemplary embodiment of the present invention.

Referring to FIG. 1, the electronic stethoscope apparatus 100-1 includes a bioacoustics sensing part 110, a noise sensing part 120, and a noise removing part 130.

The bioacoustics sensing part 110 is for sensing bioacoustics. Specifically, the bioacoustics sensing part 110 uses at least one stethoscope sensor to sense various types of bioacoustics. At this point, the bioacoustics may be at least one sound among a pulmonary sound, a heart sound, and an abdomen sound. Specially, each bioacoustics has different frequency features such as frequency bands and periods etc. For example, a heart sound may have a low frequency and a pulmonary sound may have relatively a low frequency. Also, bioacoustics sensed by the bioacoustics sensing part may be a sound of various bioacoustics combined.

According to an exemplary embodiment of the present invention, the bioacoustics sensing part may include: a stethoscope sensor (not illustrated); a sensor driving part (not illustrated); an amplifying part (not illustrated); a filtering part (not illustrated); and a A/D converting part (not illustrated). The stethoscope sensor collects bioacoustics by physical contact or physical non-contact. Here, the stethoscope sensor is a sensor configured to collect bioacoustics and convert into electrical signal, and a microphone is a typical example of it. The microphone may be a microphone for physical contact comprising an impedance matching circuit using a piezo film. The microphone may be mounted on a chest piece which makes physical contact for collecting bioacoustics.

More than two stethoscope sensors may be included to collect bioacoustics from several locations

The sensor driving part operates a stethoscope sensor and outputs a great number of bioacoustics signals that are converted into electrical signals from the stethoscope sensor. Therefore, the sensor driving part may include as many sensor driving module as corresponding to the number of the stethoscope sensor.

The amplifying part amplifies each of a plurality of bioacoustics, which are output from the sensor driving part, to reach the desired amplification gain.

The filtering part may remove a high frequency or low frequency noise from collected bioacoustics. The function of the filtering part may be performed by a noise removing part 110 which is to be described later

The A/D converting part converts and outputs the bioacoustics filtered from the filtering part or the noise removing part into digital signals.

The noise sensing part 120 senses the noise generated in the bioacoustics sensing process. The noise sensing part 120 may include a configuration similar to the mentioned bioacoustics sensing part.

That is, the noise sensing part 120 may include: a noise sensing sensor (not illustrated); a sensor driving part (not illustrated); an amplifying part (not illustrated), a filtering part (not illustrated); and an A/D converting part (not illustrated).

The noise sensing sensor is located within or outside the chest piece and may sense a noise.

The noise sensing sensor includes a microphone and may sense such noise generated by contacting with skin in the bioacoustics sensing process. The sensed sound may be output after being properly processed such as amplification, filtering or A/D conversion.

In accordance with the invention the noise sensing sensor further includes a movement sensor. The movement sensor may be located mostly in the chest piece and the movement sensor senses the movement of the chest piece in this case. For example, the movement sensor senses the vibration of the chest piece generated in the bioacoustics measuring process.

The movement sensor may be embodied as various sensors such as a gravitational acceleration sensor, a geomagnetic sensor, and a gyro sensor etc.

For example, when the movement sensor is embodied as a fluxgate geomagnetic sensor using a fluxgate, the movement sensor includes a fluxgate core consist of high permeable magnetic materials such as permalloy; an driving coil that wound with a core; and a fluxgate sensor formed with detecting coil. The number of fluxgate core may be two or three. Each fluxgate core is manufactured in mutual orthogonal form. That is, the biaxial fluxgate sensor is embodied with X-axis and Y-axis fluxgates. The triaxial fluxgate sensor is embodied with X-axis, Y-axis, and Z-axis fluxgates. Accordingly, the size and the direction of external magnetic field is measured by detecting second harmonic component that is proportional to the external magnetic field by using a detecting coil once magnetism is induced after operating signal is allowed in each operating coil where each fluxgate core is wound. Therefore, rotation angle and rotation direction may be sensed by comparing the direction of previously measured magnetic field with the direction of currently measured magnetic.

For another example, the movement sensor may include a gyro sensor. The gyro sensor is a sensor for measuring how much angle has moved in one second. That is, Coriolis force occurs when an object moves and the gyro sensor uses this formula of Coriolis force to sense angular speed that works in inertial frame. Therefore, rotation angle and rotation direction may be sensed.

Meanwhile, the movement sensor may include an acceleration sensor additionally to compensate for influence according to tilting degre. That is, the movement sensor may calculate the exact rotation angle and rotation direction by considering even a tilt angle such as a pitch angle and a roll angle that is measured by the acceleration sensor.

The noise removing part 130 removes the noise from the sensed bioacoustics and outputs it.

Specifically, when the noise sensing part 120 includes a microphone, the noise removing part 130 outputs the sensed bioacoustics by filtering with the frequency of noise signal sensed by the microphone. The microphone senses a microphone input background noise signal as a reference signal. Then, the noise removing part 130 uses the reference signal to remove the background noise coming into the stethoscope sensor with the adaptation filter.

When the noise sensing part 120 includes a movement sensor for sensing a movement of an electronic stethoscope apparatus 100-1, the noise removing part 130 uses the sensing value which is output from the movement sensor to calculate the noise signal and outputs the sensed bioacoustics by filtering it with the frequency of the noise signal. When a chest piece moves to horizontal or vertical direction and makes contact with a body, the noise is occurred accordingly and the noise sensing part 120 senses the noise. Then, the noise removing part 130 uses the movement direction and size of the bioacoustics sensing part 110 and the interrelationship among friction noise to remove the noise signal. The interrelation here means relationship of the size and the features of the noise occurred according to the movement direction and the movement size.

FIG. 2 to FIG. 5 are block diagrams illustrating a configuration of an electronic stethoscope apparatus according to various exemplary embodiments of the present invention.

Referring to FIG. 2, the electronic stethoscope apparatus 100-1according to an exemplary embodiment of the present invention includes a movement sensor which can be mounted on inner or outer surface of the chest piece. The movement sensor senses the movement of the chest piece in the stethoscope progress and outputs the movement data. The noise removing part 130 uses the movement data to estimate the noise occurring area. For an exemplary embodiment, the noise data corresponding to the accumulated movement data is stored in a storing part (not illustrated) and the measured movement data can be mapped. The noise removing part 130 outputs the sensed bioacoustics by filtering it with the frequency of the noise signal.

Referring to FIG. 3, the electronic stethoscope apparatus according to other exemplary embodiment of the present invention includes a movement sensor mounted on inner or outer surface of the chest piece, and the microphone can be located outside of the chest piece. In this case, the movement sensor senses the movement of the chest piece in the stethoscope progress and outputs the movement data. The noise removing part 130 uses the movement data to estimate the noise occurring area. The noise removing part 130 outputs the sensed bioacoustics by filtering it with the frequency of the noise signal. The microphone senses and outputs the noise generated in the bioacoustics measuring process from the outside of the chest piece. Then noise removing part 130 outputs the sensed bioacoustics by filtering it with the frequency of the sensed noise signal with the microphone.

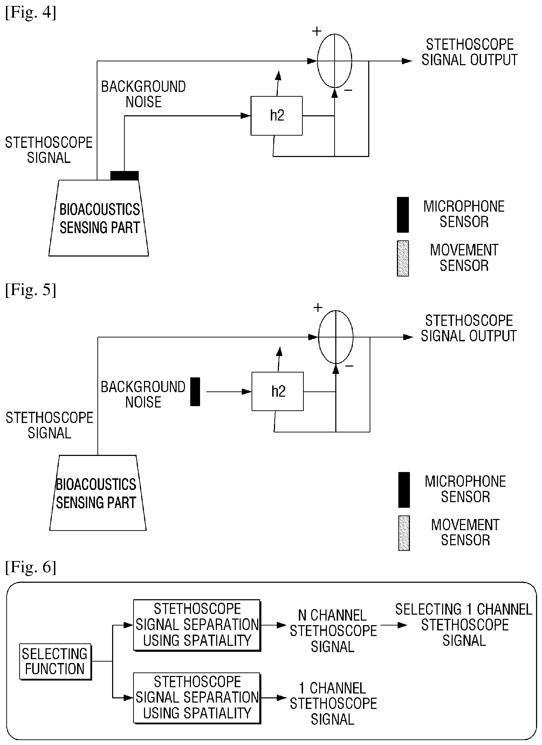

Referring to FIG. 4, the electronic stethoscope apparatus according to another exemplary embodiment of the present invention includes a microphone mounted on inner or outer surface of the chest piece. In this case the microphone senses and outputs the noise generated in the bioacoustics measuring process. Then noise removing part 130 outputs the sensed bioacoustics by filtering it with the frequency of the sensed noise signal with the microphone.

Referring to FIG. 5, the electronic stethoscope apparatus according to another exemplary embodiment of the present invention includes a microphone mounted on the outer surface of the chest piece. In this case, it is similar to the above embodiment.

Human body generates bioacoustics with different characteristics from various body parts. For example, a heart sound, a pulmonary sound, and an abdomen sound occur around a human abdomen. In this case, when locating a sensor of a stethoscope apparatus around the abdomen, the stethoscope apparatus senses bioacoustics synthesized with a heart sound, a pulmonary sound and an abdomen sound.

Especially, when a doctor uses a stethoscope apparatus to diagnose the body condition of a person, if the doctor cannot detect the exact target bioacoustics then there might be a problem of making misdiagnosis since the doctor makes a diagnosis based on the bioacoustics that is not detected properly.

Therefore, it is necessary to separate the target bioacoustics from the bioacoustics where a plurality of source bioacoustics is synthesized for a doctor to make a diagnose properly based on the bioacoustics

FIG. 6 is a reference diagram illustrating the method of separating target bioacoustics from bioacoustics where a plurality of source bioacoustics is synthesized.

Referring to FIG. 6, the target bioacoustics for diagnosis may be detected by considering the spatiality of the bioacoustics sensed by a multi stethoscope sensor (n channel stethoscope signal and separating the source bioacoustics.

Whereas, the target bioacoustics may be detected by removing reference bioacoustics sensed by the second stethoscope sensor from the bioacoustics sensed by the first stethoscope sensor.

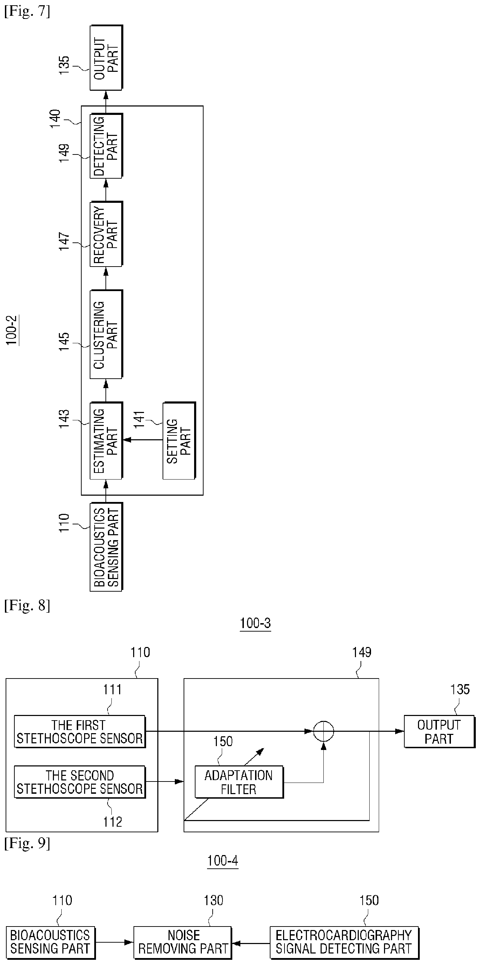

FIG. 7 is a block diagram illustrating a configuration of an electronic stethoscope apparatus 100-2 according to other exemplary embodiment, not forming part of the present invention.

Referring to FIG. 7, the electronic stethoscope apparatus 100-2 includes a bioacoustics sensing part, 110, a separation part 140, and a output part 135.

The bioacoustics sensing part 110 is already described, so the description will be omitted.

The separation part 140 uses the spatiality of the bioacoustics to separate the sensed bioacoustics into a plurality of source bioacoustics. At this point, the spatiality of the bioacoustics can be a gain and delay of bioacoustics sensed through a plurality of stethoscope sensor. That is, the separation part 140 may analyze the gain and delay of source bioacoustics and separate a plurality of source bioacoustics from the sensed bioacoustics.

The separation part 140 includes: a setting part 141; a estimating part 143; a clustering part 145; a recovery part 147; and an detecting part 149.

The setting part 141 sets a signal mixing model which is reflected with spatiality of bioacoustics. For example, the setting part 141 may set the signal mixing model as Equation 1 below when receiving bioacoustics including two source bioacoustics from two stethoscope sensors.

<Equation 1>

$$x_1(t) = s_1(1) + s_2(t)$$

$$x_1(t) = a_1 s_1(t-d_1) + a_2 s_2(t-d_2)$$

Here, x1 and x2 are signals received by the stethoscope sensor. s1 and s2 are the first source bioacoustics signal and the second source bioacoustics signal. a1 and a2 are the gain damping ratios of the first and second source bioacoustics signals. d1 and d2 are the delay values of the first and the second source bioacoustics signals.

The estimating part 143 uses the signal mixing model that is set by the setting part to estimate mixing parameter of bioacoustics signal received into each stethoscope sensor. The mixing parameter may include the gain damping ratio (a) and delay value (d).

The clustering part 145 clusters the mixing parameter estimated by the estimating part 143 in the parameter space. Specifically, the clustering part 145 may cluster the estimated mixing parameters in the parameter space having x-axis as gain damping ratio(a) and y-axis as delay value (d).

The recovery part 147 uses the mixing parameters clustered by the clustering part 145 to convert the bioacoustics into a time domain and recover a plurality of source bioacoustics. For example, the recovery part 147 may convert the signal detected in the first domain from the parameter space into the time domain and recover the signal as the first source bioacoustics, and may convert the signal detected in the second domain into the time domain and recover the signal as the second source bioacoustics.

The detecting part 149 detects the target bioacoustics among a plurality source bioacoustics recovered by the recover part 147. The target bioacoustics may be bioacoustics selected by the user but it is not limited to this, and may be defaulted in manufacturing process.

Meanwhile, the stethoscope 100-2 may further include a user input part (not illustrated) for selecting the target bioacoustics which and user wants to detect.

The output part 135 may output the detected target bioacoustics. At this point, the output part 135 may output the target bioacoustics in audio form, however, this is just an exemplary embodiment, so the target bioacoustics may be output in video form.

By the stethoscope apparatus as described above, the user may effectively separate or detect the target bioacoustics that the user wants to diagnose.

FIG. 8 is a block diagram of a configuration of an electronic stethoscope apparatus 100-3 according to another exemplary embodiment, not forming part of the present invention.

The stethoscope apparatus 100-3 includes: a bioacoustics sensing part 110 with the first stethoscope sensor 111 and the second stethoscope sensor 112; a detecting part 149; and an output part 135.

The bioacoustics sensing part 110 uses a plurality of stethoscope sensor to sense a reference bioacoustics and a synthesis bioacoustics including a reference bioacoustics and a target bioacoustics. Especially, the bioacoustics sensing part 110, as illustrated in FIG. 8, includes the first 111 and the second 112 stethoscope sensor. The bioacoustics sensing part 110 senses the synthesis bioacoustics that is synthesized with a reference bioacoustics and a target bioacoustics through the first stethoscope sensor 111, and senses the reference bioacoustics through the second stethoscope sensor 112. Meanwhile, the method in which the bioacoustics sensing part 110 uses a plurality of the stethoscope sensor for signal process is already described so the description will be omitted.

The detecting part 149 uses an adaptation filter to remove the reference bioacoustics sensed by the second stethoscope sensor 112 from the synthesis bioacoustics sensed by the first stethoscope sensor 111 and detect the target bioacoustics. Specifically, if the synthesis bioacoustics signal measured from the first stethoscope sensor 111 is input into the detecting part 149 in the state where reference bioacoustics is still mixed in, the adaptation filter of the detecting part observes the output value and changes the coefficient of the adaptation filter to perform feedback. With such operation, the adaptation filter may filter the reference bioacoustics signal from the measured synthesis bioacoustics signal and detects the target bioacoustics

The output part 135 outputs the target bioacoustics detected by the detecting part 149. The output part 135 may output the target bioacoustics in audio form; however, this is just an exemplary embodiment, so the target bioacoustics may also be output in video.

Especially, the described embodiment is useful when measuring the bioacoustics with noise accurately. For example, the pulse sound of a mother may be mixed as noise with the heart sound of a fetus when listening to the heart sound of a fetus. Therefore, the synthesis bioacoustics synthesized with the heart sound of a fetus and the pulse sound of a mother may be obtained by contacting the first stethoscope sensor 111 on the mother's abdomen. Also the pulse sound of a mother can be obtained as a reference bioacoustics by contacting the second stethoscope sensor 112 on radial artery (where you can feel pulse). With the method described above, the user may listen to the heart sound of a fetus more clearly by removing the pulse sound of a mother which is a reference bioacoustics

The stethoscope apparatus according various exemplary embodiment of the present invention enables automatic disease diagnosis through outputted stethoscope signals. In addition, the accuracy of diagnosis can be higher when combining with several medical technologies. These various exemplary embodiments are described in the following.

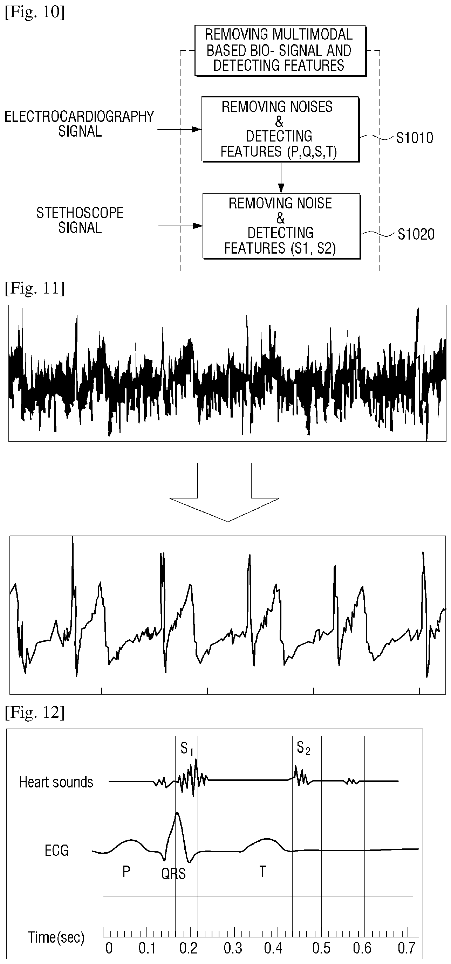

FIG. 9 is a block diagram illustrating a block diagram illustrating a configuration of an electronic stethoscope apparatus 100-4 according to another exemplary embodiment, not forming part of the present invention. FIG. 10 is a reference diagram schematically illustrating an operation of an electronic stethoscope apparatus 100-4 according to another exemplary embodiment of the present invention. FIG. 11 is a drawing illustrating a wave form of an electrocardiography signal. FIG. 12 is a drawing illustrating a location correlation between a stethoscope signal and an electrocardiography signal.

Referring to FIG. 9, the stethoscope device 100-4 includes: a bioacoustics sensing part 110; an electrocardiography signal detecting part 150; and a noise removing part 130.

The function of the bioacoustics sensing part 110 is as described above, so description is omitted

The electrocardiography signal detecting part 150 is a configuration for detecting an electrocardiography signal. Specifically, the electrocardiography signal detecting part 150 detects subtle electronic signal sensed from skin through a pair of electrodes attached on the skin when the cardiac muscle depolarizes on every heartbeat. On resting phase, each cardiac muscle cell has negative charge and it is called a membrane potential. These negative charges are decreased due to the inflow of cations such as Na+ and Ca++, so the depolarization occurs and the heart contracts. During each heartbeat, the heart provides an orderly depolarization wave form spreading out from the signal coming out from sinoatrial node to whole ventricle. A wave form of small voltage sensed by a pair of electrodes can be expressed on display in curve

Within one cycle of the electrocardiography signal, P wave, Q wave, R wave, S wave, and T wave are generally generated consecutively as illustrated in FIG. 11.

P wave indicates the contraction of an atrium and a series of P wave, R wave and S wave (ORS complex) indicates the contraction of a ventricle, and T wave is feature of the relaxation of a ventricle

The electrocardiography signal detecting part 150 detects features of the QRS complex section, P wave and T wave (S1010). The QRS complex section is an electrocardiography signal section between the QRS complex starting point and the QRS complex ending point having the point where the size of the an electrocardiography signal(voltage, y-axis) is at the peak as a center. During the process, the noise of an electrocardiography signal is removed by itself as in FIG. 10. (This is distinguished from the operation of the noise removing part which is described later.)

The noise removing part 130 uses the detected electrocardiography signal to estimate the location of the heart sound from the sensed bioacoustics. Then the noise removing part removes the noise from the sensed bioacoustics (S1020)

The noise removing part 130 may remove the noise according to the signal feature of the bioacoustics and a respiration signal. Generally, a heart sound has feature of an impulse signal, whereas, a breathing or noise sound has feature of a white noise. In addition, a heart sound and a respiration signal has different frequency band, so it is possible to detect a heart sound using a frequency filter. However, sometimes it is possible not to remove the noise completely when detecting a heart sound, so it is necessary to locate a heart sound more precisely. Since disease can be diagnosed according to the location of noise generated based on the location of a heart sound

It is possible to diagnose a disease using the above mentioned signal features of a respiration signal. A normal respiration signal has the feature of white noise and the size decreases as it goes to high frequency. However, an abnormal respiration signal includes crackle, wheezing, stridor, and pleural rub etc., and has different frequency feature with the normal respiration signal in case of diseases related to respiration organs.

As illustrated in FIG. 12, there is a correlation between the location of the stethoscope signal and the electrocardiography signal. That is, the heart sound of the stethoscope signal is located at the end part of the QRS section of the electrocardiography signal. Therefore, the noise removing part 130 uses the electrocardiography signal to locate the heart sound of the stethoscope signal. Especially, when a lot of noise is included in the stethoscope signal, it may be difficult to locate the location of the heart sound with the stethoscope signal itself. In this case, it may locate the location of the heart sound of the stethoscope signal using the electrocardiography signal.

The electronic stethoscope device 100-3 according to another exemplary embodiment, not forming part of the present invention, may remove the noise by detecting the features of the electrocardiography signal through the electrocardiography signal detecting part. Then the information is used to detect features of the stethoscope signal. That is, it may be used to remove the noise from the stethoscope signal and detect accurate features of a heart sound or a pulmonary sound.

When auscultating a respiration signal, the noise removing part 130 filters the heart sound from the detected bioacoustics after estimating the location of the heart sound through the location of the features

The electronic stethoscope apparatus similar to the above mentioned exemplary embodiment may be further considered. An electronic stethoscope apparatus with a purse wave detecting means is described in the following.

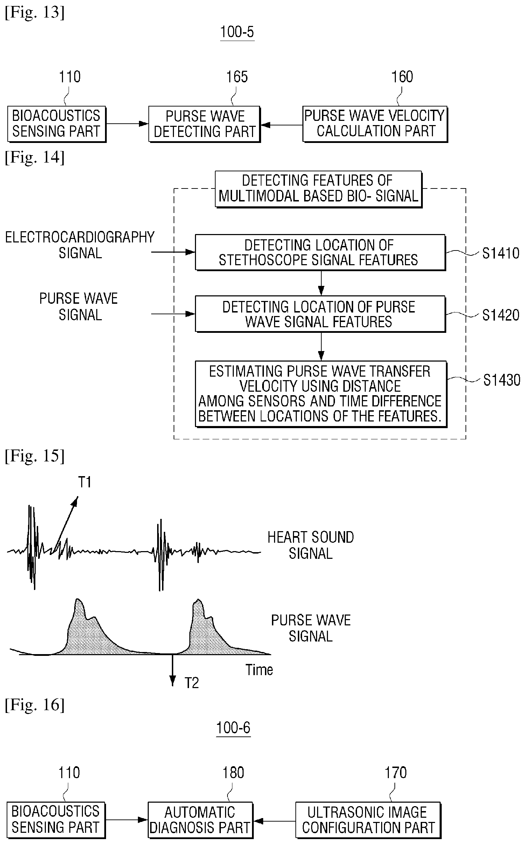

FIG. 13 is a block diagram illustrating a configuration of an electronic stethoscope apparatus 100-5 according to another exemplary embodiment of the present invention. FIG. 14 is a reference diagram schematically illustrating an operation of an electronic stethoscope apparatus 100-5 according to another exemplary embodiment of the present invention. FIG. 15 is a drawing illustrating wave forms of a heart sound and a pulse wave.

Referring to FIG. 13, the stethoscope apparatus 100-3 includes: a bioacoustics sensing part 110; a pulse wave signal detecting part 160; and a pulse wave velocity calculating part 165.

The bioacoustics sensing part 110, as mentioned above, outputs the stethoscope signal. As mentioned above, the noise filtering is possible from the sensed bioacoustics and features of the stethoscope signal can be detected (S1410).

The pulse wave signal detecting part 160 detects a pulse wave signal. The pulse wave signal detecting part 160 measures the pulse wave generated as a result of the repetition of the contraction and relaxation of a human heart from the blood vessel, and the pulse wave signal detecting part is configured with the sensor elements having several physical features according to the measuring purpose. For example, a pressure sensor using elements such as piezzo element generating an output signal according to the pressure may be used. The pulse wave signal detecting part 160 detects the pulse wave signal and calculates the feature location (S1420).

The pulse wave velocity calculating part 165 uses the time between the first location of the sensed bioacoustics and the second location of the detected pulse purse wave signal to estimate the pulse wave transfer velocity (S1430).

Referring to FIG. 15, start location time (T1) of the heart sound is estimated from the exemplary embodiment of the present invention and the time (T2) of the incisura(v point) location is measured from the pulse wave. Then it may estimate the pulse wave transfer velocity by using both distance between the bioacoustics sensing part 110 and the pulse wave signal detecting part 160 and time difference between the start location time(T1) of the heart sound and the time(T2) of the incisura (v point) location.

In case the pulse wave transfer velocity is slow, the blood vessel can be diagnosed as healthy.

Since the wave of the pulse wave is absorbed and the pulse wave transfer velocity slows down in case of having soft and elastic blood vessel. The pulse wave transfer velocity also slows down when having a big internal diameter of the blood vessel.

The electronic stethoscope apparatus with ultrasonic detecting means is described in the following.

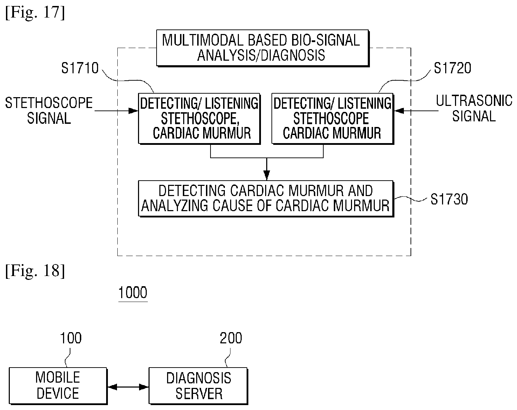

FIG. 16 is a block diagram illustrating an operation of an electronic stethoscope apparatus 100-6 according to another exemplary embodiment not forming part of the present invention. FIG. 17 is a reference diagram schematically illustrating an operation of an electronic stethoscope apparatus 100-6.

Referring to FIG. 16, the electronic stethoscope apparatus 100-6 includes: a bioacoustics sensing part 110; an ultrasonic image configuration part 170; and an automatic diagnosis part 180.

The bioacoustics sensing part 110 outputs the stethoscope signal (S1710). As mentioned above, the noise filtering is possible from the sensed bioacoustics.

The ultrasonic image configuration part 170 irradiates the ultrasonic signal onto a body part and senses the ultrasonic echo signal reflected from the body part. For this, the ultrasonic image configuration part includes a probe included of a transducer with a vibrator; and a tuning coil for sensing the ultrasonic echo signal. In addition, the ultrasonic image configuration part includes an A/D converter for processing received signal and a signal processing part.

The ultrasonic image configuration part 170 receives the ultrasonic signal reflected from the body part and generates the B-mode image having a black-and-white signal or the C-mode (color mode) image.

The B-mode(Brightness-mode) image is an image configuring the body part where the ultrasonic is irradiated in black and white by using the ultrasonic echo signal reflected from the body part. When having the distance to the body part on the horizontal axis and the amplitude of the reflected echo on the vertical axis, the amplitude can be marked by replacing it with the brightness of the dots. B-mode can be configured by using this method in black and white image.

On the other hand, C- mode(color Doppler mode) image is an image configuring the body part where ultrasonic is irradiated in color by using the reflected ultrasonic echo signal. In case the ultrasonic echo signal is received and then the frequency deviation is occurred by the Doppler Effect, the ultrasonic image configuration part 170 may measure the velocity of blow flow by calculating the frequency deviation. Then the C-mode image can be configured using the same

The automatic diagnosis part 180 performs automatic diagnosis by using the ultrasonic image and the bioacoustics signal. Specifically, the automatic diagnosis part 180 performs automatic diagnosis by analyzing the heart sound and the ultrasonic image which is a cause of heart sound (S1730). For this, parameters on various diseases are stored in advance and managed, and the disease matching the parameters of ultrasonic image and bioacoustics signal is searched. Not only ultrasonic image, but also the ultrasonic sound converted from the ultrasonic image can be used.

A remote diagnosis system 1000 according to various exemplary embodiment of the present invention is described in the following.

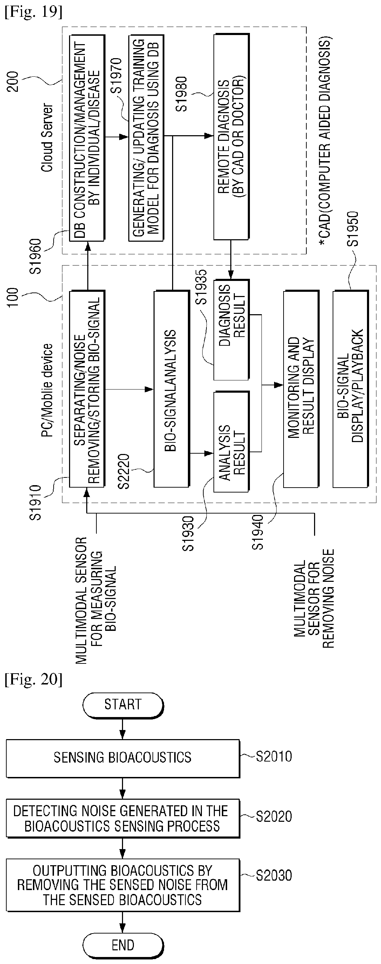

FIG. 18 is a block diagram illustrating a configuration of a remote diagnosis system 1000. FIG. 19 is a flowchart illustrating a remote diagnosis method.

Referring to FIG. 18, the remote diagnosis system 1000 includes a mobile device 100 and a diagnosis server 200.

The mobile device 100 detects or measures various bio-signals (S1910). The mobile device 100 removes and stores noise with above mentioned various method Then the mobile device 100 analyzes the bio-signal (S1920). The result of the diagnosis can be displayed solely (S1930). However, the result can be displayed with the result of the diagnosis by the server 200 (S1940). The diagnosis server 200 builds and manages the database by individual! by diseases after receiving bio-signal from the mobile device 100 (S1960). Then the diagnosis server generates and updates a training model for diagnosis using the DB (S1970). The training model is used in automatic diagnosis.

The remote diagnosis can be performed by a doctor, or by an automatic diagnosis of the diagnosis server 200. The result of the diagnosis is transmitted to the mobile device 100 (S1935). The mobile device 100 displays the result of the diagnosis.

As mentioned above, abnormal respiration signal can be detected through stethoscope signal. Therefore, it is possible to diagnosis diseases such as bronchitis, pulmonary edema, cardiac insufficiency, pneumonia, pulmonary infarction, and asthma etc. In addition, it is possible to diagnosis cardiac murmur, arrhythmia, and heart rate etc. related to heart diseases.

With electrocardiography, it is possible to diagnose atrial fibrillation, atrial flutter, ventricular tachycardia, myocardial infarction, ischemic heart disease, and valvular heart disease (causing abnormality of blood flow) through heart rate and arrhythmia.

Arrhythmia, heart rate, Heart valve stenosis, and obstruction etc. maybe diagnosed through ultrasonic image or sound, and it is also possible to diagnose the condition of the blood vessel. That is, valvular heart disease, stenosis and obstruction causing abnormality of blood flow can be diagnosed.

Arrhythmia and heart rate can be diagnosed through the purse wave, and it is possible to diagnose vein aging, vein elasticity, blood circulation age, artery disorder, and peripheral vascular disorder etc

The automatic diagnosis method according to various exemplary embodiments of the present invention is described in the following.

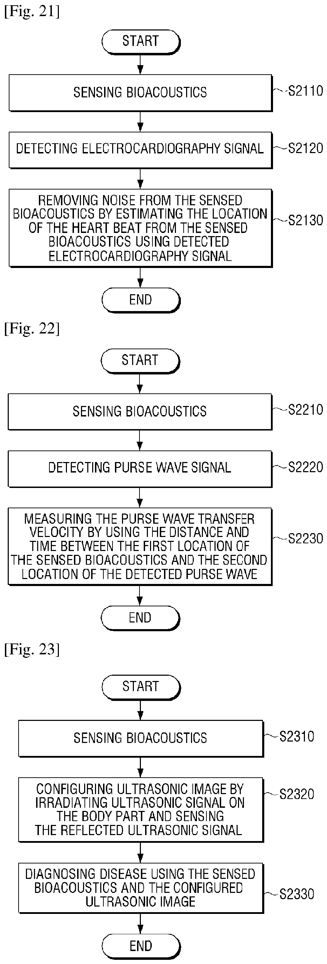

FIG. 20 to FIG. 23 are flowcharts of an automatic diagnostic method.

The automatic diagnosis method includes the steps of sensing bioacoustics (S2010); sensing noise generated in the bioacoustics sensing process (S2020); and outputting bioacoustics by removing the sensed noise from the sensed bioacoustics (S2030).

The noise sensing step may perform using a microphone.

In addition, the microphone is mounted on the chest piece of the electronic stethoscope apparatus, and the noise removing step may output the sensed bioacoustics by filtering with the frequency of the noised signal sensed through the microphone.

Furthermore, the noise sensing step uses a movement sensor for sensing movement of the electronic stethoscope apparatus to sense the noise, and the noise removing step uses the sensing value which is output from the movement sensor to calculate the noise signal and outputs the sensed bioacoustics by filtering it with the frequency of the noise signal.

The automatic diagnosis method according to other exemplary embodiment not forming part of the present invention includes the steps of: sensing bioacoustics (S2110); detecting electrocardiography signal (S2120); and estimating the location of the heart sound from the sensed bioacoustics by using the detected electrocardiography signal and of removing the noise from the sensed bioacoustics (S2130).

At this point, the electrocardiography signal detecting step detects the features of the detected electrocardiography signal, and the noise removing step uses the feature of the detected electrocardiography signal to estimate the location of a heart sound from the sensed bioacoustics.

The automatic diagnosis method according to another exemplary embodiment not forming part of the present invention includes the steps of sensing bioacoustics (S2210); detecting a pulse wave signal (S2220); and measuring the pulse wave transfer velocity by using the distance and time between the first location of the sensed bioacoustics and the second location of the detected purse wave signal (S2230).

The automatic diagnosis method according to another exemplary embodiment not forming part of the present invention includes the steps of sensing bioacoustics (S2310); configuring an ultrasonic image by irradiating an ultrasonic signal onto a body part and sensing the reflected ultrasonic signal (S2320); and diagnosing disease by using the sensed bioacoustics and the configured ultrasonic image (S2330).

Meanwhile, the above mentioned automatic diagnosis method can be stored in nontemporary recording media which is possible to be read on computer as a form of program. The nontemporary readable media is not a media storing data for a brief moment such as a register, cache etc., but a media storing data semipermanently and a media that is readable by electronic devices. For example, CD, DVD, hard drive, Blu-ray disk, USB, memory card, ROM

Those skilled in the art will appreciate that various modifications and additions are possible, without departing from the scope of the invention as defined in the appended claims:

**Claims**

1. An electronic stethoscope apparatus (100-1) comprising:

    a chest piece;
    a bioacoustics sensing part (110) for sensing bioacoustics;
    a noise sensing part (120) for sensing noise generated in the bioacoustics sensing process, the noise sensing part (120) comprising a movement sensor for sensing movement of the electronic stethoscope apparatus (100-1), the movement sensor being mounted on an inner or outer surface of the chest piece; and
    a noise removing part (130) for removing the sensed noise and outputting the sensed bioacoustics with the removed noise,
    wherein the noise removing part (130) is operable to use the sensed movement to calculate a noise signal, and is operable to output the sensed bioacoustics by filtering the sensed bioacoustics with the frequency of the noise signal;
    the noise sensing part further comprises a microphone; and
    noise removing part (130) is further operable to output the sensed bioacoustics by filtering the sensed bioacoustics with a frequency of a noise signal sensed by the microphone in addition to filtering the sensed bioacoustics with the frequency of the noise signal calculated from the sensed movement, and
    the noise removing part (130) is configured to use the sensed movement to estimate the noise occurring area.

2. The electronic stethoscope apparatus (100-1) according to claim 1, wherein the bioacoustics sensing part (110) comprises a further microphone, with the microphone of the bioacoustics sensing part (110) mounted on the chest piece.

3. The electronic stethoscope apparatus (100-1) according to claim 2, wherein the microphone of the noise sensing part (120) is mounted on the chest piece.

4. A method of using an electronic stethoscope apparatus, the method comprising:

    sensing a bioacoustics (S2010);
    sensing a noise generated in the bioacoustics sensing process (S2020), the noise being sensed by a movement sensor for sensing movement of the electronic stethoscope apparatus (100-1), the movement sensor being mounted on an inner or outer surface of a chest piece of the electronic stethoscope apparatus (100-1); and

by means of a noise removing part (130) of the electronic stethoscope apparatus, removing the sensed noise from the sensed bioacoustics and outputting the sensed bioacoustics with the removed noise (S2030),

wherein a noise signal is calculated by the noise removing part (130) using the sensed movement, and the sensed bioacoustics are outputted by the noise removing part (130) after filtering the sensed bioacoustics with the frequency of the noise signal calculated from the sensed movement; and

a further noise sensing step is performed by using a microphone of a noise sensing part (120) and wherein the noise removing part (130) outputs the sensed bioacoustics by filtering the sensed bioacoustics with a frequency of a noise signal sensed by the microphone of the noise sensing part (120) in addition to filtering the sensed bioacoustics with the frequency of the noise signal calculated from the sensed movement,

wherein the noise removing step uses the sensed movement to estimate the noise occurring area.

5. The method according to claim 4, wherein the wherein the bioacoustics sensing part (110) comprises a further microphone mounted on the chest piece.

**Patentansprüche**

1. Elektronische Stethoskopvorrichtung (100-1), umfassend:

ein Bruststück;

einen Bioakustik-Erfassungsteil (110) zum Erfassen von Bioakustik;

einen Rauscherfassungsteil (120) zum Erfassen von Rauschen, das in dem bioakustischen Erfassungsprozess erzeugt wird, wobei der Rauscherfassungsteil (120) einen Bewegungssensor zum Erfassen einer Bewegung der elektronischen Stethoskopvorrichtung (100-1) umfasst, wobei der Bewegungssensor an einer Innen- oder Außenfläche des Bruststücks angebracht ist;

einen Rauschentfernungsteil (130) zum Entfernen des erfassten Rauschens und zum Ausgeben der erfassten Bioakustik mit dem entfernten Rauschen,

wobei das Rauschentfernungsteil (130) betreibbar ist, um die erfasste Bewegung zu verwenden, um ein Rauschsignal zu berechnen, und betreibbar ist, um die erfasste Bioakustik auszugeben, indem die erfasste Bioakustik mit der Frequenz des Rauschsignals gefiltert wird;

der Rauscherfassungsteil ferner ein Mikrofon umfasst; und

der Rauschentfernungsteil (130) ferner betreibbar ist, um die erfasste Bioakustik durch Filtern der erfassten Bioakustik mit einer Frequenz eines durch das Mikrofon erfassten Rauschsignals zusätzlich zum Filtern der erfassten Bioakustik mit der Frequenz des aus der erfassten Bewegung berechneten Rauschsignals auszugeben, und

der Rauschentfernungsteil (130) so konfiguriert ist, dass er die erfasste Bewegung verwendet, um den Bereich mit auftretendem Rauschen zu schätzen.

2. Elektronische Stethoskopvorrichtung (100-1) nach Anspruch 1, wobei der Bioakustik-Erfassungsteil (110) ein weiteres Mikrofon umfasst, wobei das Mikrofon des Bioakustik-Erfassungsteils (110) an dem Bruststück angebracht ist.

3. Elektronische Stethoskopvorrichtung (100-1) nach Anspruch 2, wobei das Mikrofon des Rauscherfassungsteils (120) an dem Bruststück angebracht ist.

4. Verfahren zur Verwendung eines elektronischen Stethoskopgeräts, wobei das Verfahren umfasst:

Erfassen einer Bioakustik (S2010);

Erfassen eines im bioakustischen Erfassungsprozess erzeugten Rauschens (S2020), wobei das Rauschen von einem Bewegungssensor zum Erfassen einer Bewegung der elektronischen Stethoskopvorrichtung (100-1) erfasst wird, wobei der Bewegungssensor an einer inneren oder äußeren Oberfläche eines Bruststücks des elektronischen Stethoskopgeräts (100-1) angebracht ist; und

mittels eines Rauschentfernungsteils (130) der elektronischen Stethoskopvorrichtung, Entfernen des erfassten Rauschens aus der erfassten Bioakustik und Ausgeben der erfassten Bioakustik mit dem entfernten Rauschen (S2030), wobei ein Rauschsignal von dem Rauschentfernungsteil (130) unter Verwendung der erfassten Bewegung berechnet wird und die erfasste Bioakustik von dem Rauschentfernungsteil (130) ausgegeben wird, nachdem die erfasste Bioakustik mit der aus der erfassten Bewegung berechneten Frequenz des Rauschsignals gefiltert wurde; und

ein weiterer Rauscherfassungsschritt unter Verwendung eines Mikrofons eines Rauscherfassungsteils (120) durchgeführt wird, und wobei der Rauschentfernungsteil (130) die erfasste Bioakustik ausgibt, indem er die erfasste Bioakustik mit einer Frequenz eines Rauschsignals, das durch das Mikrofon des Rauscherfassungsteils (120) erfasst wird, zusätzlich zum Filtern der erfassten Bioakustik mit der aus der erfassten Bewegung berechneten Frequenz des Rauschsignals filtert,
wobei der Rauschentfernungsschritt die erfasste Bewegung verwendet, um den Bereich mit auftretendem Rauschen abzuschätzen.

5. Verfahren nach Anspruch 4, wobei das Bioakustik-Erfassungsteil (110) ein weiteres Mikrofon umfasst, das an dem Bruststück angebracht ist.

**Revendications**

1. Appareil stéthoscope électronique (100-1) comprenant :

une pièce de poitrine ;
une partie de détection bioacoustique (110) pour détecter la bioacoustique ;
une partie de détection de bruit (120) pour détecter le bruit généré dans le processus de détection bioacoustique, la partie de détection de bruit (120) comprenant un capteur de mouvement pour détecter le mouvement de l'appareil stéthoscope électronique (100-1), le capteur de mouvement étant monté sur un ou la surface externe de la pièce de poitrine ; et
une partie de suppression de bruit (130) pour supprimer le bruit détecté et émettre la bioacoustique détectée avec le bruit supprimé,
dans lequel la partie de suppression de bruit (130) peut fonctionner pour utiliser le mouvement détecté pour calculer un signal de bruit, et peut fonctionner pour émettre la bioacoustique détectée en filtrant la bioacoustique détectée avec la fréquence du signal de bruit ;
la partie de détection de bruit comprend en outre un microphone ; et
une partie de suppression de bruit (130) peut en outre fonctionner pour émettre la bioacoustique détectée en filtrant la bioacoustique détectée avec une fréquence d'un signal de bruit détecté par le microphone en plus de filtrer la bioacoustique détectée avec la fréquence du signal de bruit calculé à partir du mouvement détecté, et la partie de suppression de bruit (130) est configurée pour utiliser le mouvement détecté pour estimer la zone d'apparition de bruit.

2. Appareil stéthoscope électronique (100-1) selon la revendication 1, dans lequel la partie de détection bioacoustique (110) comprend un autre microphone, le microphone de la partie de détection bioacoustique (110) étant monté sur la pièce de poitrine.

3. Appareil stéthoscope électronique (100-1) selon la revendication 2, dans lequel le microphone de la partie de détection de bruit (120) est monté sur la pièce de poitrine.

4. Procédé d'utilisation d'un appareil stéthoscope électronique, le procédé comprenant :

la détection d'une bioacoustique (S2010) ;
la détection d'un bruit généré dans le processus de détection bioacoustique (S2020), le bruit étant détecté par un capteur de mouvement pour détecter le mouvement de l'appareil stéthoscope électronique (100-1), le capteur de mouvement étant monté sur une surface interne ou externe d'une pièce de poitrine de l'appareil stéthoscope électronique (100-1) ; et
au moyen d'une partie de suppression de bruit (130) de l'appareil stéthoscope électronique, la suppression du bruit détecté de la bioacoustique détectée et l'émission de la bioacoustique détectée avec le bruit supprimé (S2030),
dans lequel un signal de bruit est calculé par la partie de suppression de bruit (130) à l'aide du mouvement détecté, et la bioacoustique détectée est émise par la partie de suppression de bruit (130) après filtrage de la bioacoustique détectée avec la fréquence du signal de bruit calculé à partir du mouvement détecté ; et
une autre étape de détection de bruit est exécutée à l'aide d'un microphone d'une partie de détection de bruit (120) et dans lequel la partie de suppression de bruit (130) émet la bioacoustique détectée en filtrant la bioacoustique détectée avec une fréquence d'un signal de bruit détecté par le microphone de la partie de détection de bruit (120) en plus du filtrage de la bioacoustique détectée avec la fréquence du signal de bruit calculée à partir du mouvement détecté,
dans lequel l'étape de suppression de bruit utilise le mouvement détecté pour estimer la zone d'apparition de bruit.

5. Procédé selon la revendication 4, dans lequel la partie de détection bioacoustique (110) comprend un autre microphone monté sur la pièce de poitrine.

[Fig. 1]

100-1

110   130   120

BIOACOUSTICS SENSING PART → NOISE REMOVING PART ← NOISE SENSING PART

[Fig. 2]

STETHOSCOPE SIGNAL OUTPUT

BACKGROUND NOISE

h2

STETHOSCOPE SIGNAL

BIOACOUSTICS SENSING PART

VIBRATION SIGNAL

h1

■ MICROPHONE SENSOR

▒ MOVEMENT SENSOR

[Fig. 3]

STETHOSCOPE SIGNAL OUTPUT

BACKGROUND NOISE

h2

STETHOSCOPE SIGNAL

BIOACOUSTICS SENSING PART

VIBRATION SIGNAL

h1

■ MICROPHONE SENSOR

▒ MOVEMENT SENSOR

[Fig. 4]

[Fig. 5]

[Fig. 6]

[Fig. 7]

100-2

BIOACOUSTICS SENSING PART — 110

ESTIMATING PART — 143

SETTING PART — 141

CLUSTERING PART — 145

RECOVERY PART — 147

DETECTING PART — 149 / 140

OUTPUT PART — 135

[Fig. 8]

100-3

110

111 — THE FIRST STETHOSCOPE SENSOR

112 — THE SECOND STETHOSCOPE SENSOR

150 — ADAPTATION FILTER

149

135 — OUTPUT PART

[Fig. 9]

100-4

110 — BIOACOUSTICS SENSING PART

130 — NOISE REMOVING PART

150 — ELECTROCARDIOGRAPHY SIGNAL DETECTING PART

[Fig. 10]

```
┌─────────────────────────────┐
│  REMOVING MULTIMODAL        │
│  BASED BIO- SIGNAL AND      │
│  DETECTING FEATURES         │
└─────────────────────────────┘
```

ELECTROCARDIOGRAPHY SIGNAL → 

```
┌─────────────────────────────┐
│  REMOVING NOISES            │
│  &                          │
│  DETECTING                  │    S1010
│  FEATURES (P,Q,S,T)         │
└─────────────────────────────┘
```

STETHOSCOPE SIGNAL → 

```
┌─────────────────────────────┐
│  REMOVING NOISE             │
│  &                          │
│  DETECTING                  │    S1020
│  FEATURES (S1, S2)          │
└─────────────────────────────┘
```

[Fig. 11]

[Fig. 12]

[Fig. 13]

100-5

110              165              160

| BIOACOUSTICS SENSING PART | → | PURSE WAVE DETECTING PART | ← | PURSE WAVE VELOCITY CALCULATION PART |

[Fig. 14]

DETECTING FEATURES OF MULTIMODAL BASED BIO- SIGNAL

ELECTROCARDIOGRAPHY SIGNAL →

DETECTING LOCATION OF STETHOSCOPE SIGNAL FEATURES    S1410

PURSE WAVE SIGNAL →

DETECTING LOCATION OF PURSE WAVE SIGNAL FEATURES    S1420

ESTIMATING PURSE WAVE TRANSFER VELOCITY USING DISTANCE AMONG SENSORS AND TIME DIFFERENCE BETWEEN LOCATIONS OF THE FEATURES.    S1430

[Fig. 15]

T1

HEART SOUND SIGNAL

PURSE WAVE SIGNAL

Time

T2

[Fig. 16]

100-6

110              180              170

| BIOACOUSTICS SENSING PART | → | AUTOMATIC DIAGNOSIS PART | ← | ULTRASONIC IMAGE CONFIGURATION PART |

[Fig. 17]

MULTIMODAL BASED BIO-SIGNAL
ANALYSIS/DIAGNOSIS

S1710

STETHOSCOPE
SIGNAL

DETECTING/ LISTENING
STETHOSCOPE,
CARDIAC MURMUR

DETECTING/ LISTENING
STETHOSCOPE
CARDIAC MURMUR

S1720

ULTRASONIC
SIGNAL

DETECTING CARDIAC MURMUR AND
ANALYZING CAUSE OF CARDIAC MURMUR

S1730

[Fig. 18]

1000

100

MOBILE
DEVICE

200

DIAGNOSIS
SERVER

[Fig. 19]

[Fig. 20]

[Fig. 21]

```
                    ┌──────────┐
                    │  START   │
                    └────┬─────┘
                         ▼
      ┌─────────────────────────────────────┐
      │       SENSING BIOACOUSTICS          │──S2110
      └─────────────────┬───────────────────┘
                         ▼
      ┌─────────────────────────────────────┐
      │ DETECTING ELECTROCARDIOGRAPHY SIGNAL│──S2120
      └─────────────────┬───────────────────┘
                         ▼
      ┌─────────────────────────────────────┐
      │  REMOVING NOISE FROM THE SENSED     │
      │ BIOACOUSTICS BY ESTIMATING THE      │
      │ LOCATION OF THE HEART BEAT FROM     │──S2130
      │ THE SENSED BIOACOUSTICS USING       │
      │ DETECTED ELECTROCARDIOGRAPHY SIGNAL │
      └─────────────────┬───────────────────┘
                         ▼
                    ┌──────────┐
                    │   END    │
                    └──────────┘
```

[Fig. 22]

```
                    ┌──────────┐
                    │  START   │
                    └────┬─────┘
                         ▼
      ┌─────────────────────────────────────┐
      │       SENSING BIOACOUSTICS          │──S2210
      └─────────────────┬───────────────────┘
                         ▼
      ┌─────────────────────────────────────┐
      │     DETECTING PURSE WAVE SIGNAL     │──S2220
      └─────────────────┬───────────────────┘
                         ▼
      ┌─────────────────────────────────────┐
      │  MEASURING THE PURSE WAVE TRANSFER  │
      │ VELOCITY BY USING THE DISTANCE AND  │
      │ TIME BETWEEN THE FIRST LOCATION OF  │──S2230
      │ THE SENSED BIOACOUSTICS AND THE     │
      │ SECOND LOCATION OF THE DETECTED     │
      │ PURSE WAVE                          │
      └─────────────────┬───────────────────┘
                         ▼
                    ┌──────────┐
                    │   END    │
                    └──────────┘
```

[Fig. 23]

```
                    ┌──────────┐
                    │  START   │
                    └────┬─────┘
                         ▼
      ┌─────────────────────────────────────┐
      │       SENSING BIOACOUSTICS          │──S2310
      └─────────────────┬───────────────────┘
                         ▼
      ┌─────────────────────────────────────┐
      │  CONFIGURING ULTRASONIC IMAGE BY    │
      │ IRRADIATING ULTRASONIC SIGNAL ON    │──S2320
      │ THE BODY PART AND SENSING           │
      │ THE REFLECTED ULTRASONIC SIGNAL     │
      └─────────────────┬───────────────────┘
                         ▼
      ┌─────────────────────────────────────┐
      │ DIAGNOSING DISEASE USING THE SENSED │
      │ BIOACOUSTICS AND THE CONFIGURED     │──S2330
      │ ULTRASONIC IMAGE                    │
      └─────────────────┬───────────────────┘
                         ▼
                    ┌──────────┐
                    │   END    │
                    └──────────┘
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006062704 A1